# EUROPÄISCHE PATENTSCHRIFT

(11) **EP 0 460 465 B1**
(45) Veröffentlichungstag und Bekanntmachung des Hinweises auf die Patenterteilung: **04.12.1996**
(21) Anmeldenummer: 91108429.1
(22) Anmeldetag: 24.05.1991
(51) Int. Cl.: C07B 37/02, C07C 2/04, C07C 15/44, C07C 17/26, C07C 25/24

(54) **Verfahren zur Herstellung von an der Doppelbindung mit Ethylen verlängerten Styrol-Derivaten mit in der gebildeten Verlängerungskette verbleibender Doppelbindung und neue mit Ethylen verlängerte Styrol-Derivate**
Process for the preparation of styrene derivatives having a double bond in an ethylene-extended chain and ethylene-extended styrene derivatives
Procédé pour la préparation de dérivés de styrène ayant une liaison double dans une chaîne allongée par l'éthylène et des dérivés de styrène allongés par l'éthylène

(30) Priorität: 06.06.1990 DE 4018068
(43) Veröffentlichungstag der Anmeldung: 11.12.1991
(73) Patentinhaber: BAYER AG, 51368 Leverkusen (DE)
(72) Erfinder: Ostoja Starzewski, Karl-Heinz Aleksander, Dr., W-6368 Bad Vilbel (DE)

(56) Entgegenhaltungen:
- EP-A- 0 192 845
- EP-A- 0 219 092
- EP-A- 0 235 714
- EP-A- 0 291 795
- US-A- 4 956 123
- JOURNAL OF THE AMERICAN CHEMICAL SOCIETY. Bd. 100, Nr. 7, 29. März 1978, GASTON, PA, US, Seiten 2181-2190, A. PADWA et al.

## Beschreibung

Die Erfindung betrifft ein Verfahren zur Umsetzung von Styrol-Derivaten mit Ethylen in Gegenwart eines Nickel-Katalysators, der einen Phosphor-Sauerstoff-Chelatliganden trägt, wobei Styrol-Derivate erhalten werden, die an der olefinischen Doppelbindung mit Ethylen verlängert sind und bei denen in der gebildeten Verlängerungskette eine Doppelbindung verbleibt.

Solche mit Ethylen verlängerten Styrol-Derivate sind interessante Zwischenprodukte, die sich wegen der in der Verlängerungskette verbleibenden Doppelbindung als Vorstufe für Pfropfpolymerisate, beispielsweise mit Methylmethacrylat oder Maleinsäureanhydrid, eignen oder mit denen sich polymeranaloge Umsetzungen durchführen lassen. Das erfindungsgemäß eingesetzte Styrol-Derivat kann darüber hinaus die weiter unten genannten Substituenten tragen, die weitere Umsetzungen ermöglichen bzw. weitere Eigenschaften in ein Polymer einbringen. Besonders interessant sind erfindungsgemäß erhältliche Produkte, in denen das Styrol-Derivat eine weitere Vinylgruppe trägt, also beispielsweise Divinylbenzol ist. In diesem Fall wird überwiegend nur eine Vinylgruppe verlängert. Die dabei gebildeten verlängerten Styrol-Derivate sind bifunktionell; sie tragen zwei verschieden reaktive olefinische Doppelbindungen. Die nicht verlängerte Vinylgruppe kann sodann in an sich bekannter Weise zu Styrol-analogen Homo- oder Copolymerisationen genutzt werden. Die daraus hervorgehenden Polymere tragen weiterhin ihre über den Aromaten gebundene Poly(oligo)ethylen-Seitenketten aus der erfindungsgemäßen Verlängerung und sind somit Poly(oligo)ethylen-modifiziert. An den Doppelbindungen dieser Seitenketten können dann Pfropfreaktionen, Derivatisierungen, Vernetzungen und andere Reaktionen durchgeführt werden.

EP-A 219 092 beschreibt ein Verfahren zur Herstellung von Alkenylaromaten Ar-C(CH₃, R¹)-CH=CHR² durch Umsetzung von Vinylaromaten Ar-C(R¹)=CH₂ mit α-Olefinen H₂C=CHR², in denen R² Wasserstoff oder C₁-C₁₀-Alkyl ist. Die Kettenlänge des Alkenylsubstituenten ist abhängig von der Kettenlänge des umgesetzten α-Olefins. Das Katalysatorsystem für diese Umsetzung besteht aus a) einem Nickel(II)-salz einer Carbonsäure, einem Ni-alkoholatt oder -phenolat, b) einem Phosphit oder Phosphin und c) einer aluminiumorganischen Verbindung, Vinyl-α-butyl-styrol ist im Rahmen einer allgemeinen Formel in US 4 956 123 angegeben, ein Herstellungsverfahren findet sich dort nicht.

Es wurde ein Verfahren zur Herstellung von Mischungen von Verbindungen der Formel 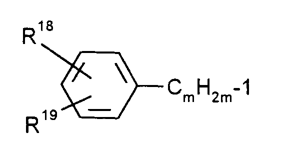 worin
- R¹⁸: Wasserstoff, C₁-C₄-Alkyl, C₂-C₄-Alkenyl, C₂-C₇-Acyl, Fluor, Chlor oder Brom,
- R¹⁹: Wasserstoff, C₁-C₄-Alkyl, Vinyl oder Chlor und
- m: 4 bis 204 bedeuten,
durch Umsetzung einer Verbindung der Formel 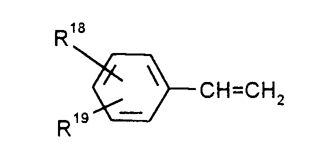 worin
- R¹⁸ und R¹⁹: die oben definierte Bedeutung besitzen,
mit Ethylen in Gegenwart eines Nickel-Katalysators, der einen Phosphor-Sauerstoff-Liganden trägt und die Struktureinheit 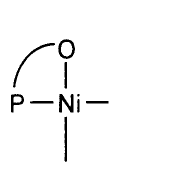 enthält, bei einer Temperatur von 20 bis 160°C und einem Ethylendruck von 1 bis 200 bar gefunden.

Eine größere Anzahl von Nickel-Katalysatoren, die einen Phosphor-Sauerstoff-Chelatliganden tragen und die erfindungsgemäß einsetzbar sind, sind dem Fachmann bekannt.

In bevorzugter Weise wird in Gegenwart eines Nickel-Katalysators gearbeitet, der herstellbar ist durch Umsetzung einer Nickel-(O)-Verbindung oder einer Verbindung, die in situ in eine Nickel-(O)-Verbindung übergeführt werden kann, mit einer Phosphorverbindung der Formel 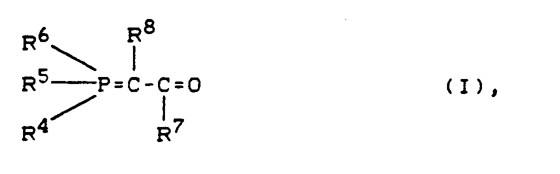 worin
- R⁴, R⁵, R⁶, R⁷ und R⁸: unabhängig voneinander geradkettiges oder verzweigtes C₁-C₂₀-Alkyl, C₂-C₂₀-Alkenyl, C₁-C₂₀-Alkoxy, C₃-C₈-Cycloalkyl, C₆-C₁₂-Aryl, C₆-C₁₂-Aryloxy, C₇-C₁₅-Aralkyl oder C₇-C₁₅-Aralkoxy bedeuten, wobei
- R⁷: zusätzlich Wasserstoff und
- R⁸: zusätzlich Wasserstoff, Acyl oder Sulfonat bedeuten können
oder Nickel-Katalysatoren, herstellbar durch Umsetzung einer Nickel-(O)-Verbindung oder einer Verbindung, die in situ in eine Nickel-(O)-Verbindung übergeführt werden kann, mit einem Addukt aus einer chinoiden Verbindung oder Maleinsäureanhydrid und einem Phosphin der Formel 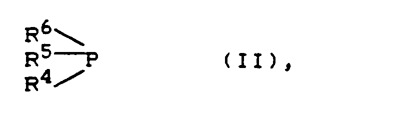 in der
- R⁴, R⁵ und R⁶: die genannte Bedeutung haben.

Solche Phosphor-ylid-Nickel-Verbindungen können sowohl einzeln als auch als Gemisch mehrerer von ihnen eingesetzt werden.

In bevorzugter Weise hat R⁴ die Bedeutung von gegebenenfalls substituierten C₆-C₁₂-Aryl.

In weiterhin bevorzugter Weise wird bei der Herstellung der obigen Katalysatoren von einer Nickel-(O)-Verbindung oder einer Verbindung, die in situ in eine Nickel-(O)-Verbindung übergeführt werden kann, einer Verbindung der Formel (I) und zusätzlich einer Verbindung der Formel 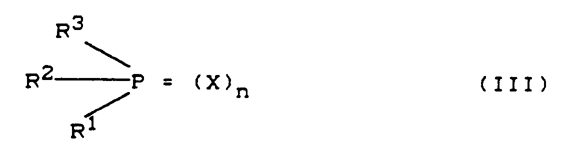 ausgegangen, worin
- R¹, R² und R³: unabhängig voneinander geradkettiges oder verzweigtes C₁-C₂₀-Alkyl, C₁-C₂₀-Alkoxy, C₃-C₈-Cycloalkyl, C₂-C₂₀-Alkenyl, Di-(C₁-C₄-alkyl)-amino, C₆-C₁₂-Aryl, C₆-C₁₂-Aryloxy, C₇-C₁₅-Aralkyl oder C₇-C₁₅-Aralkoxy bedeuten,
- X: doppelt gebundenen Sauerstoff, die doppelt gebundene Gruppe NR⁹ oder die doppelt gebundene Gruppe 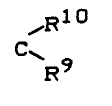 bedeutet, wobei
R⁹ und R¹⁰ unabhängig voneinander Wasserstoff, Silyl, Acyl, Chlorphenyl, Nitrophenyl, C₁-C₆-Alkylphenyl, Cyano, Phenyl-C₂-C₆-alkenyl oder R¹ bedeuten, und
- n: den Wert Null oder Eins annimmt.

Auch bei der Herstellung der obigen Katalysatoren, ausgehend von einer Nickel-(O)-Verbindung oder einer Verbindung, die in situ in eine Nickel-(O)-Verbindung übergeführt werden kann, und einem Addukt aus einer chinoiden Verbindung oder Maleinsäureanhydrid und einer Verbindung der Formel (II), ist es bevorzugt, zusätzlich noch von einer Verbindung der Formel (III) auszugehen.

In besonders bevorzugter Weise wird in Gegenwart eines Nickel-Katalysators gearbeitet, der erhalten wird durch Umsetzung einer Nickel-(O)-Verbindung mit Phosphorverbindungen der Formeln 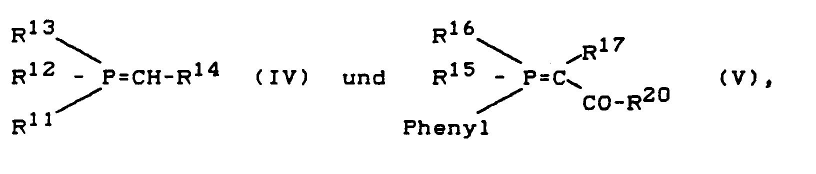 worin
- R¹¹, R¹² und R¹³: unabhängig voneinander C₁-C₈-Alkyl, Phenyl oder Benzyl bedeuten,
- R¹⁴: für Wasserstoff, C₁-C₈-Alkyl oder Phenyl steht,
- R¹⁵, R¹⁶ und R¹⁷: unabhängig voneinander C₁-C₈-Alkyl oder Phenyl bedeuten, wobei R¹⁷ zusätzlich Wasserstoff oder Acyl bedeuten kann, und
- R²⁰: Phenyl oder C₁-C₄-Alkyl bedeutet.
oder eines Nickel-Katalysators, herstellbar durch Umsetzung einer Nickel-(O)-Verbindung oder einer Verbindung, die in situ in eine Nickel-(O)-Verbindung übergeführt werden kann, mit einem Addukt aus Benzochinon oder Maleinsäureanhydrid und einem Phosphin der Formel 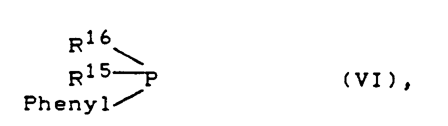 in der
- R¹⁵ und R¹⁶: die genannte Bedeutung haben,
und einer Verbindung der Formel (IV).

In bevorzugter Weise bedeutet R²⁰ Phenyl.

Zur Herstellung des Katalysators werden pro Mol Nickel-(O)-Verbindung 0 bis 4 Mol der Verbindung der Formel (III) und 1 bis 4 Mol der Verbindung der Formel (I), bevorzugt pro Mol der Nickel-(O)-Verbindung etwa 1 Mol der Verbindung der Formel (III) oder (IV) und etwa 1 Mol der Verbindung der Formel (I) bzw. (V) eingesetzt. Gleiche molare Verhältnisse gelten, wenn anstelle einer Verbindung der Formel (I) bzw. (V) ein Chinon/Phosphin-Addukt oder ein Maleinsäureanhydrid/ Phosphin-Addukt der beschriebenen Art tritt.

Die Temperatur zur Herstellung des Katalysators beträgt 0 bis 100°C, bevorzugt 20 bis 70°C. Bei der Herstellung wird unter Ausschluß von Sauerstoff, vorzugsweise in einem Lösungsmittel, das gegenüber den Reaktanden inert sein muß, wie Benzol, Toluol, Cyclohexan oder n-Hexan, gearbeitet. Nach seiner Herstellung wird der Katalysator als Feststoff gewöhnlich durch Filtrieren isoliert, wobei man nach Bedarf die Lösung zuvor einengt und/oder abkühlt. Der Katalysator kann jedoch auch ohne Isolierung, d.h. als Lösung oder Suspension, direkt zur erfindungsgemäßen Umsetzung eingesetzt werden.

Als Nickel-(O)-Verbindungen seien beispielhaft Ni(cyclooctadien)₂ und Ni(allyl)₂ genannt. Als Nickelverbindungen, die in situ in Nickel-(O)-Verbindungen übergeführt werden können, seien beispielsweise genannt: Ni-acetylacetonat, Ni-octanoat und Ni-stearat, die mit Hilfe von üblichen Reduktionsmitteln, wie Boranat, Alanat, Aluminiumalkylen oder Lithiumorganylen, reduziert werden können.

Als Alkyl, bevorzugt C₁-C₈-Alkyl, das geradkettig oder verzweigt sein kann, sei beispielsweise genannt: Methyl, Ethyl, Propyl, Isopropyl, Butyl, Isobutyl, tert.-Butyl, die isomeren Pentyle, Hexyle, Octyle, Decyle, Dodecyle, Hexadecyle und Eicosyle. Besonders bevorzugtes Alkyl hat 1 bis 4 C-Atome.

Als C₁-C₂₀-Alkoxy, das geradkettig oder verzweigt sein kann, sei beispielsweise genannt: Methoxy, Ethoxy, Propoxy, Isopropoxy, Butoxy, Isobutoxy, tert.-Butoxy, die isomeren Pentyloxy, Hexyloxy, Octyloxy, Decyloxy, Dodecyloxy, Eicosyloxy. Bevorzugtes Alkoxy hat 1 bis 8 C-Atome, besonders bevorzugtes 1 bis 4 C-Atome.

Als C₃-C₈-Cycloalkyl sei beispielsweise genannt: Cyclopropyl, Cyclobutyl, Cyclopentyl, Cyclohexyl, Methyl-cyclopentyl, Methyl-cyclohexyl, Cycloheptyl, Cyclooctyl.

Als C₆-C₁₂-Aryl sei beispielsweise genannt: Phenyl, Naphthyl, Biphenylyl. Bevorzugtes Aryl ist Phenyl.

Als C₂-C₂₀-Alkenyl sei beispielsweise genannt: Vinyl, Propenyl, Allyl, Butenyl, Pentenyl, Hexenyl, Octenyl, Decenyl oder Eicosenyl sowie deren verzweigte Isomere.

Als C₆-C₁₂-Aryloxy sei beispielsweise genannt: Phenoxy, Naphthyloxy, Biphenyloxy. Bevorzugt ist Phenoxy.

Als C₇-C₁₅-Aralkyl sei beispielsweise genannt: Benzyl, Phenylethyl, Phenylpropyl, Naphthyl-methyl, bevorzugt Benzyl.

Als C₇-C₁₅-Aralkoxy sei beispielsweise genannt: Benzyloxy, Phenyl-ethyloxy, Phenyl-propyloxy, Naphthyl-methyloxy, bevorzugt Benzyloxy.

Als Di-(C₁-C₄-alkyl)-amino sei beispielsweise genannt: Dimethylamino, Diethylamino, Dipropylamino, Methylbutylamino, Ethyl-butylamino u.a.

Als Silyl sei beispielsweise Tri-C₁-C₄-alkyl-silyl, Triphenylsilyl oder gemischte trisubstituierte Alkylphenyl-silyle, bevorzugt Tri-C₁-C₄-alkyl-silyle, wie Trimethylsilyl, Triethylsilyl und andere genannt.

Als Acyl sei C₁-C₈-Alkylcarbonyl oder C₆-C₁₂-Arylcarbonyl genannt, die in der unten genannten Weise substituiert sein können, wie Acetyl, Propionyl, Butyryl, C₅-Alkyl-carbonyl, C₈-Alkyl-carbonyl, Benzoyl, substituiertes Benzoyl oder Naphthyl-carbonyl. Bevorzugtes Acyl ist substituiertes oder nicht substituiertes C₁-C₄-Alkylcarbonyl oder Benzoyl. Besonders bevorzugt sind Acetyl oder Benzoyl.

Die genannten Substituenten können ein- bis dreifach, bevorzugt ein- oder zweifach, besonders bevorzugt einfach, durch C₁-C₄-Alkyl, durch C₁-C₄-Alkoxy, durch C₆-C₁₂-Aryl, durch C₆-C₁₂-Aryloxy oder Nitro, bevorzugt durch C₁-C₄-Alkyl, durch C₁-C₄-Alkoxy, durch Phenyl oder Phenoxy substituiert sein, wobei bei mehrfacher Substitution die Substituenten verschiedene aus der genannten Aufzählung sein können. In diesem Sinne wird beispielsweise Tolyl als Aryl mitverstanden.

Als chinoide Verbindungen kommen o- oder p-chinoide Verbindungen der Benzol- und Naphthalin-Reihe sowie Anthrachinone in Frage, die noch in der oben beschriebenen Weise substituiert sein können Beispielhaft seien p-Benzochinon, 1,4-Naphthochinon und 9,10-Anthrachinon genannt.

Die bevorzugten Nickel-Verbindungen mit Phosphor-Sauerstoff-Chelatliganden stehen nach den vorliegenden Erkenntnissen in Einklang mit der Formel 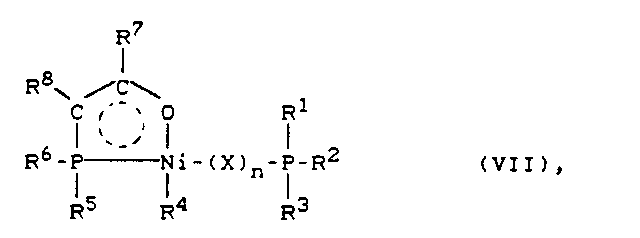 worin
- X, n und R¹ bis R⁸: die vorstehend genannten Bedeutungen besitzen.

Für den Fall, daß anstelle einer Verbindung der Formel (I) beispielsweise ein Maleinsäureanhydrid/Phosphin-Addukt der beschriebenen Art tritt, wird aus der Formel (VII) die nachstehende Formel

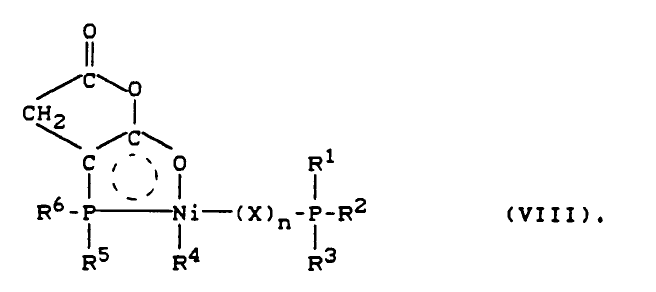

Allgemeines Strukturmerkmal der erfindungsgemäß einsetzbaren Nickel-Katalysatoren, die einen Phosphor-Sauerstoff-Chelatliganden tragen, ist folgende Konfiguration

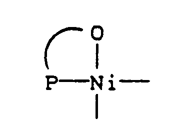

Bevorzugte Reste R¹, R² und R³ sind C₁-C₈-Alkyl, Cyclohexyl, Phenyl, Tolyl, Benzyl, Di-(C₁-C₄-alkyl)-amino, Phenoxy und C₁-C₄-Alkoxy.

R⁴ ist vorzugsweise C₆-C₁₂-Aryl, besonders bevorzugt Phenyl.

R⁵, R⁶, R⁷ und R⁸ sind unabhängig voneinander vorzugsweise Cyclohexyl, Phenyl, Tolyl, Benzyl, Vinyl und C₁-C₄-Alkyl.

R⁷ ist darüber hinaus bevorzugt Wasserstoff oder C₁-C₄-Alkoxy, R⁸ darüber hinaus bevorzugt Wasserstoff, Acetyl, Benzoyl oder die Sulfonatgruppe.

R⁹ und R¹⁰ sind vorzugsweise Wasserstoff, C₁-C₈-Alkyl, Phenyl, Chlorphenyl, Nitrophenyl, C₁-C₆-Alkylphenyl, Trimethylsilyl, Cyano, C₂-C₆-Alkenyl und Phenyl-C₂-C₆-alkenyl.

Zur erfindungsgemäßen Umsetzung werden 0,01 bis 100 mmol Nickel-Katalysator pro Mol Styrol-Derivat, bevorzugt 0,1 bis 10 mmol Nickel-Katalysator, besonders bevorzugt 0,2 bis 5 mmol, Nickel-Katalysator eingesetzt. Es ist weiterhin möglich, diese Nickel-Katalysatoren durch Aluminiumorganyle, bevorzugt Alkyl- oder Alkoxy-aluminiumverbindungen zu aktivieren.

Das erfindungsgemäße Verfahren wird bei einer Temperatur von 20 bis 160°C, bevorzugt bei 30 bis 140°C, besonders bevorzugt bei 40 bis 120°C, ganz besonders bevorzugt bei 50 bis 100°C, durchgeführt.

Es wird bei einem Ethylen-Druck von 1 bis 200 bar, bevorzugt 2 bis 50 bar, besonders bevorzugt 3 bis 25 bar, gearbeitet.

Als Styrol-Derivat wird erfindungsgemäß eines der Formel 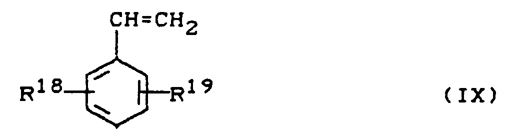 eingesetzt, in der
- R¹⁸: Wasserstoff, C₁-C₄-Alkyl, C₂-C₄-Alkenyl, C₂-C₇-Acyl, Fluor, Chlor oder Brom und
- R¹⁹: Wasserstoff, C₁-C₄-Alkyl, Vinyl oder Chlor bedeuten.

C₂-C₇-Acyl ist beispielsweise Acetyl, Propionyl, Butyl, Benzoyl, bevorzugt Benzoyl.

In bevorzugter Weise werden Styrol-Derivate der Formel 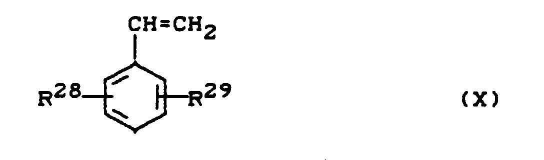 eingesetzt, in der
- R²⁸: Wasserstoff, Methyl, Ethyl, i-Butyl, C₂-C₇-Acyl, Vinyl oder Chlor und
- R²⁹: Wasserstoff, Vinyl, Methyl oder Chlor bedeuten.

In besonders bevorzugter Weise werden Styrol oder Divinylbenzol eingesetzt.

Die erfindungsgemäß einzusetzenden Styrol-Derivate können sowohl in reiner Form als auch als technisches Gemisch eingesetzt werden. Ein wichtiges Beispiel für diesen letzteren Fall ist technisches Divinylbenzol, das noch Ethyl-styrol und/oder Diethyl-benzol enthält. Weiterhin sei Benzoyl-(z.B. 3-Benzoyl-)styrol genannt.

Das erfindungsgemäße Verfahren wird in der flüssigen Phase durchgeführt. Hierbei kann grundsätzlich ohne Mitverwendung eines inerten Lösungsmittels gearbeitet werden, wenn das Styrol-Derivat flüssig ist.

In vielen Fällen wird das erfindungsgemäße Verfahren in Gegenwart eines inerten Lösungsmittels durchgeführt. Als solche inerten Lösungsmittel kommen beispielsweise in Frage: n-Hexan, Cyclohexan, Petrolether, Ligroin, Benzol, Toluol, Chlorbenzol, Aceton, Dimethylformamid und andere unter den Reaktionsbedingungen nicht angreifbare Lösungsmittel, bevorzugt Cyclohexan oder Toluol.

Das inerte Lösungsmittel wird in einer Gewichtsmenge, die das 0,1- bis 100-fache, bevorzugt das 0,5- bis 20-fache, bezogen auf das Styrol-Derivat, beträgt, eingesetzt.

Das Umsetzungsprodukt des erfindungsgemäßen Verfahrens stellt im allgemeinen zunächst eine homologe Reihe von verlängerten Styrol-Derivaten dar, bei denen die Verlängerung aus einem Molekül Ethylen pro Molekül des zugrunde liegenden Styrol-Derivats bzw. aus zwei oder mehr Molekülen Ethylen pro Mol des zugrunde liegenden Styrol-Derivats besteht und bei denen in der Verlängerungskette eine Doppelbindung verbleibt. Die Einzelkomponenten der Styrol-Ethylen-Umsetzung können durch die folgenden Formeln dargestellt werden:

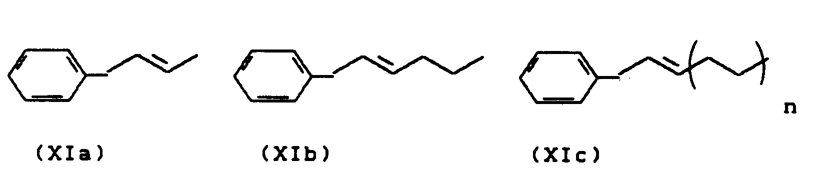

Formel (XIa) stellt hierbei den Fall Styrol:Ethylen = 1:1 dar, Formel (XIb) stellt den Fall Styrol:Ethylen = 1:2 dar, und Formel (XIc) stellt den allgemeinen Fall Styrol:Ethylen = 1:1 + n dar, wobei n hauptsächlich Werte von 0 (Null) bis 100, insbesondere 0 bis 30, ganz besonders 0-10 annimmt.

Infolge von Isomerisierungsreaktionen werden im erfindungsgemäßen Verfahren auch homologe Reihen von isomeren Produkten gebildet, die mit den folgenden Formeln in Übereinstimmung zu stehen scheinen:

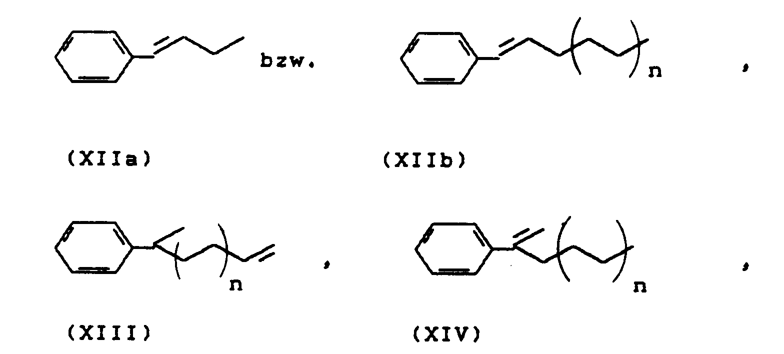

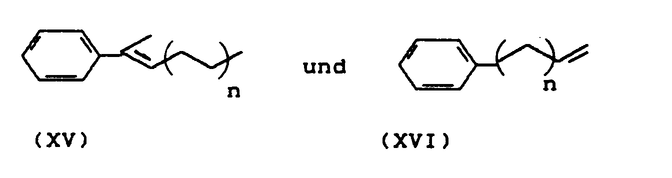

Aufgrund der dem Fachmann bekannten cis-trans-Isomerie bei olefinisch ungesättigten Verbindungen treten in Ergänzung zu den oben als trans-Isomere dargestellten Verbindungen der Formeln (XI), (XII) und (XV) stets auch die korrespondierenden cis-Isomere auf.

In den Formeln (XI a-c), (XII a und b) und (XIII) bis (XVII) bedeuten die Knick- und Endpunkte der geknickten Linie (= Verlängerungskette) in einer dem Fachmann geläufigen Weise C-Atome, die mit der erforderlichen Zahl von H-Atomen besetzt sind. Dadurch ergibt sich beispielsweise für die Formeln (XIa) und (XIII) folgende ausführliche Schreibweise

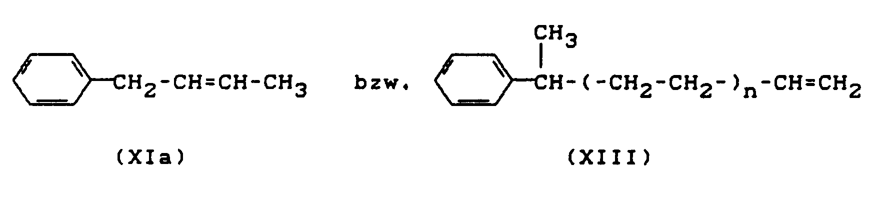

In den Formeln (XI) bis (XVII) wurde der Übersichtlichkeit halber die Kernsubstitution gemäß Formel (IX) bzw. (X) weggelassen.

Die Gesamtheit des Verfahrensproduktes des erfindungsgemäßen Verfahrens mit einer in der verzweigten oder nicht verzweigten Verlängerungskette verbleibenden Doppelbindung läßt sich somit durch die Formel 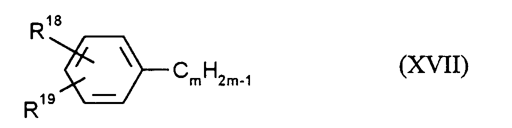 darstellen, in der der Index m den Wert des obigen Index 2n+4 annimmt und R¹⁸ und R¹⁹ den obigen Bedeutungsumfang haben.

In der oben dargestellten Weise handelt es sich im allgemeinen um das Gemisch der unter die Formel (XVII) fallenden Homologen.

Als Nebenprodukte treten Polyolefine auf.

Ein solches Produktgemisch kann in einer dem Fachmann bekannten Weise, beispielsweise durch chromatografische Trennung, fraktionierte Destillation oder Fällung in Einzelkomponenten oder in Fraktionen aufgetrennt werden.

Das erfindungsgemäße Verfahren ist insofern überraschend, als mit dem einzusetzenden Nickel-Katalysator, mit dem bekanntlich Ethylen polymerisiert werden kann, hier nunmehr die Polyethylen-Bildung zur Nebenreaktion wird. Auch die Polystyrol-Bildung wird nahezu völlig unterdrückt. Die homologe Reihe der Styrol/Ethylen-Kopplungsprodukte wird zum hauptsächlichen Reaktionsprodukt, wobei eine Verschiebung zu höheren oder niedrigeren Molekulargewichten nach den bekannten Methoden für die Polyethylen-Molekulargewichtssteuerung gelingt. Bevorzugt sind Molmassen unter 10 000 g/mol, besonders bevorzugt unter 1000 g/mol.

Für das erfindungsgemäße Verfahren eignen sich z.B. folgende Verfahrensweisen:
a) Vorlegen des festen, suspendierten oder gelösten Katalysators (oder seiner Komponenten), Zugabe der Monomeren bei der gewünschten Temperatur gleichzeitig oder nacheinander;
b) Vorlegen der Monomeren, Injektion der Katalysator-Lösung oder -Suspension (oder seiner Komponenten) bei der gewünschten Temperatur, gegebenenfalls mit anschließendem Erhitzen;
c) kontinuierliches Dosieren der Katalysator-Lösung (oder seiner Komponenten) und der Monomeren bei vorgegebenen gewünschten Polymerisationsbedingungen (Druck, Temperatur).

Das erfindungsgemäße Verfahren kann beispielsweise wie folgt durchgeführt werden: In einem Autoklav wird das Lösungsmittel vorgelegt. Danach wird die vorgesehene Menge an Styrol-Derivat zugesetzt. Sodann wird Ethylen allein oder im Gemisch mit einem Inertgas bis zum gewünschten Reaktionsdruck in den verschlossenen Autoklaven eingepumpt, gegebenenfalls unter Berücksichtigung des Druckanstiegs bei Reaktionstemperatur. Sodann wird der Autoklav auf die gewünschte Reaktionstemperatur erwärmt und der Nickel-Katalysator als Feststoff, als Suspension oder als Lösung zugesetzt. Vorzugsweise wird eine Katalysatorlösung simultan zum Ethylenverbrauch zugepumpt (Multi-Puls-Verfahren). Die Durchführung der polymerisierenden Kopplung des Styrols wird durch Schütteln des Autoklaven oder durch eine geeignete Hub-oder Rühreinrichtung unterstützt. Das Ethylen kann im Maße seines Verbrauchs während der Umsetzung ergänzt werden. Nach Beendigung der Reaktion wird der Autoklav abgekühlt, entspannt und geöffnet. Das Reaktionsgemisch wird beispielsweise destillativ aufgearbeitet. Hierbei werden zunächst das gegebenenfalls mitverwendete inerte Lösungsmittel und das nicht umgesetzte Styrol-Derivat, beispielsweise durch Destillation, abgetrennt. Das verbleibende Umsetzungsgemisch mit den erfindungsgemäß hergestellten verlängerten Styrol-Derivaten kann sodann durch Feindestillation, durch Kristallisation, Fällung oder durch andere Trennoperationen in Einzelkomponenten oder in geeignete Fraktionen aufgetrennt werden. Bei allen Destillationen wird vorteilhafterweise in Gegenwart von üblichen Stabilisatoren gearbeitet, um eine thermische Polymerisation zu unterdrücken.

Eine Reihe von erfindungsgemäß herstellbaren mit Ethylen verlängerten Styrol-Derivaten mit in der gebildeten Verlängerungskette verbleibender Doppelbindung sind neu.

Die Erfindung betrifft daher weiterhin Mischungen von mit Ethylen verlängerten Styrol-Derivaten. Es handelt sich um Mischungen von Verbindungen der Formel 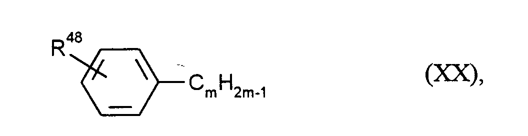 worin
- R⁴⁸: Vinyl oder Benzoyl und
- m: eine Zahl von 4 bis 204, bevorzugt 4 bis 64 bedeuten
mit Ausnahme von Buten-(2)-yl-styrol, Buten-(3)-yl-styrol, Hexen-(2)-yl-styrol und Verbindungen, in denen R⁴⁸ Benzoyl bedeutet und m den Wert 4 annimmt.

Die neuen mit Ethylen verlängerten Styrol-Derivate der Formel (XX) stellen das Gemisch der homologen Reihen dar.

Ebenso umfassen die genannten neuen Stoffe auch das Gemisch mit den technischen Begleitstoffen der zugrundeliegenden Styrole und/oder mit den homologen, mit Ethylen verlängerten Umsetzungsprodukten für den Fall, daß von in technischer Reinheit vorliegenden Styrol-Derivaten ausgegangen wird; dieser Fall ist weiter oben für technisches Divinylbenzol erläutert worden.

### Beispiel 1

### Katalysatorherstellung [NiPh(Ph₂PCHCMeO)(Prⁱ₃PCHPh)]

40 mmol Bis-cyclooctadien-nickel(O) in 250 ml trockenem argongesättigten Toluol wurden unter Argon mit 40 mmol Acetylmethylen-triphenylphosphoran und 40 mmol Triisopropyl-phosphinbenzyliden gemischt. Unter intensivem Rühren erwärmte man ca. 3 Stunden auf 60°C. Die dunkelbraune Reaktionsmischung wurde unter Argon filtriert und das Filtrat im Vakuum bis zur Trockne eingeengt. Der so erhaltene Roh-Katalysator wurde bei 60°C in Toluol gelost, mit Hexan bis zur bleibenden Trübung versetzt und in der Kälte zur Kristallisation gebracht, durch Schlenk-Filtration isoliert, mit Hexan gewaschen und im Vakuum getrocknet.

### Beispiele 2 - 10

### Allgemeine Versuchsdurchführung

In einem Autoklaven geeigneter Größe wurden die in den folgenden Tabellen genannte Menge an Toluol, die genannte Menge an Katalysator und die genannte Menge an Styrol-Derivat vorgelegt. Sodann wurde eine solche Menge Ethylen aufgedrückt, daß bei der angegebenen Reaktionstemperatur der in den Tabellen angegebene Ethylendruck erreicht wurde. Während der in den Tabellen angegebenen Reaktionszeit wurde Ethylen zur Aufrechterhaltung des angegebenen Druckes nachgeführt. Durch Auswiegen des Rückstands nach destillativer Abtrennung von Lösungsmittel und nicht umgesetztem Styrol-Derivat wurde die Ausbeute ermittelt. Die Tabellen zeigen die durch Gaschromatographie erhaltenen Ergebnisse bezüglich der prozentualen Verteilung an verlängerten Styrol-Derivaten (bezeichnet als "Produkt") und mitentstandenem α-Olefin (Polyethylen-Nebenprodukt).

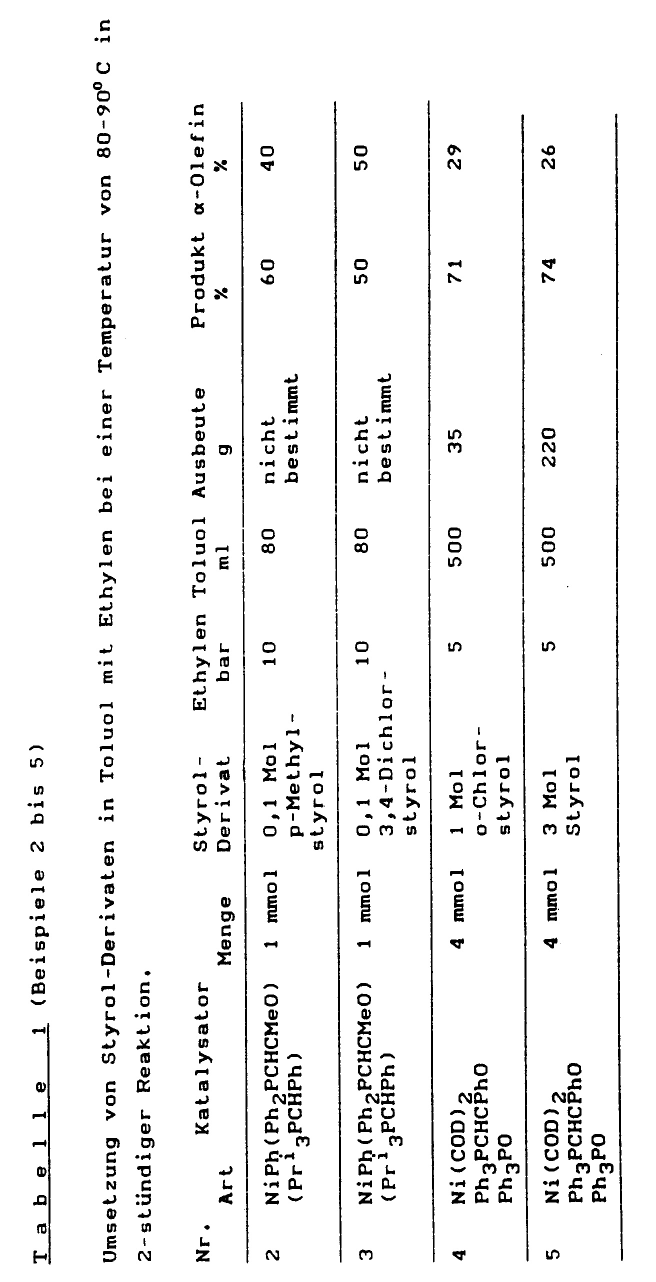

**Tabelle 2**

| (Beispiele 6 und 7) Umsetzung von Divinylbenzol (DVB, frisch destilliert, jeweils 390 g) in Toluol (jeweils 1000 ml) mit Ethylen in 2-stündiger Reaktion; Katalysator-Dosierung durch Multi-Puls | | | | | | | |
|---|---|---|---|---|---|---|---|
| Nr. | Katalysator | | Ethylen bar | Temp. °C | Ausbeute g | Produkt % | α-Olefin % |
| | Art | Menge | | | | | |
| 6 | Ni(COD)₂ | 4 mmol in situ in 150 ml Toluol | 2,5 | 90 | 173 | 89 | 11 |
| | Ph₃PCHCPhO | | | | | | |
| | Ph₃PO | | | | | | |
| 7 | NiPh(Ph₂PCHCPhO) | 4 mmol isoliert in 150 ml Toluol | 5,0 | 50 | 278 | 85 | 15 |
| | (Ph₃P) | | | | | | |
| zum Katalysator: COD = Cyclooctadien; Ph = Phenyl; Me = Methyl Produkt = Gesamtmenge der erhaltenen verlängerten Styrol-Derivate | | | | | | | |

**Tabelle 3**

| (Beispiele 8 bis 10) Umsetzung von Divinylbenzol (DVB, frisch destilliert, jeweils 390 g) in Toluol (jeweils 1000 ml) mit Ethylen bei 90°C in 2-stündiger Reaktion mit 4 mmol Ni(COD)₂/Ph₃PCHCPhO/Ph₃PNSiMe₃ als in situ-Katalysator in 150 ml Toluol, Katalysator-Dosierung: Multi-Puls | | | | |
|---|---|---|---|---|
| Nr. | Ethylen bar | Ausbeute g | Produkt % | α-Olefin % |
| 8 | 10 | 320 | 70 | 30 |
| 9 | 5 | 345 | 83 | 17 |
| 10 | 2,5 | 110 | 91 | 9 |

## Patentansprüche

1. Verfahren zur Herstellung von Mischungen von Verbindungen der Formel 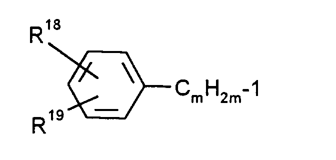 worin
R¹⁸ Wasserstoff, C₁-C₄-Alkyl, C₂-C₄-Alkenyl, C₂-C₇-Acyl, Fluor, Chlor oder Brom,
R¹⁹ Wasserstoff, C₁-C₄-Alkyl, Vinyl oder Chlor und
m 4 bis 204 bedeuten,
durch Umsetzung einer Verbindung der Formel 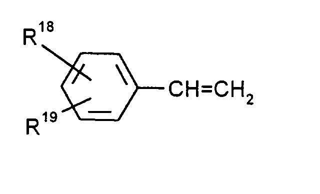 worin
R¹⁸ und R¹⁹ die oben definierte Bedeutung besitzen,
mit Ethylen in Gegenwart eines Nickel-Katalysators, der einen Phosphor-Sauerstoff-Liganden trägt und die Struktureinheit 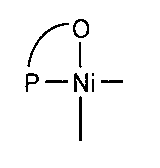 enthält, bei einer Temperatur von 20 bis 160°C und einem Ethylendruck von 1 bis 200 bar.

2. Verfahren nach Anspruch 1, dadurch gekennzeichnet, daß man in Gegenwart eines Nickel-Katalysators arbeitet, der erhalten wird durch Umsetzung einer Nickel-(0)-Verbindung oder einer Verbindung, die in situ in eine Nickel-(0)-Verbindung übergeführt werden kann, mit einer Phosphorverbindung der Formel 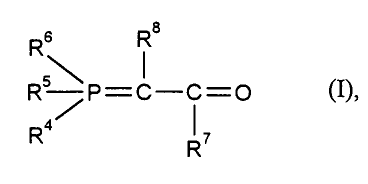 worin
R⁴, R⁵, R⁶, R⁷ und R⁸ unabhängig voneinander geradkettiges oder verzweigtes C₁-C₂₀-Alkyl, C₂-C₂₀-Alkenyl, C₁-C₂₀-Alkoxy, C₃-C₈-Cycloalkyl, C₆-C₁₂-Aryl, C₆-C₁₂-Aryloxy, C₇-C₁₅-Aralkyl oder C₇-C₁₅-Aralkoxy bedeuten, wobei
R⁷ zusätzlich Wasserstoff und
R⁸ zusätzlich Wasserstoff, Acyl oder Sulfonat bedeuten können
oder Nickel-Katalysatoren, herstellbar durch Umsetzung einer Nickel-(0)-Verbindung oder einer Verbindung, die in situ in eine Nickel-(0)-Verbindung übergeführt werden kann, mit einem Addukt aus einer chinoiden Verbindung oder Maleinsäureanhydrid und einem Phosphin der Formel 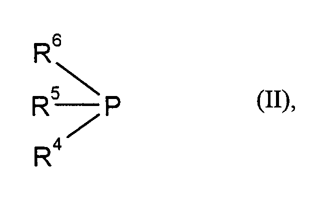 in der
R⁴, R⁵ und R⁶ die genannte Bedeutung haben.

3. Verfahren nach Anspruch 2, dadurch gekennzeichnet, daß zur Herstellung des Nickel-Katalysators aus einer Nickel-(O)-Verbindung oder einer Verbindung, die in situ in eine Nickel-(O)-Verbindung übergeführt werden kann, und aus einer Verbindung der Formel (I) oder aus einem Addukt aus einer chinoiden Verbindung oder Maleinsäureanhydrid und einer Verbindung der Formel (II) zusätzlich von einer Verbindung der Formel 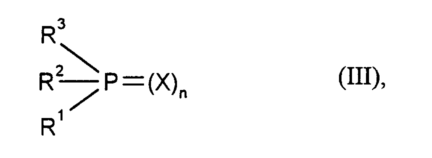 ausgegangen wird, worin
R¹, R² und R³ unabhängig voneinander geradkettiges oder verzweigtes C₁-C₂₀-Alkyl, C₁-C₂₀-Alkoxy, C₃-C₈-Cycloalkyl, C₂-C₂₀-Alkenyl, Di-(C₁-C₄-alkyl)-amino, C₆-C₁₂-Aryl, C₆-C₁₂-Aryloxy, C₇-C₁₅-Aralkyl oder C₇-C₁₅-Aralkoxy bedeuten,
X doppelt gebundenen Sauerstoff, die doppelt gebundene Gruppe NR⁹ oder die doppelt gebundene Gruppe 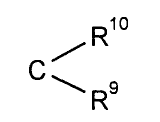 bedeutet, wobei
R⁹ und R¹⁰ unabhängig voneinander Wasserstoff, Silyl, Acyl, Chlorphenyl, Nitrophenyl, C₁-C₆-Alkylphenyl, Cyano, Phenyl-C₂-C₆-alkenyl oder R¹ bedeuten, und
n den Wert Null oder Eins annimmt.

4. Verfahren nach Anspruch 3, dadurch gekennzeichnet, daß man in Gegenwart eines Nickel-Katalysators arbeitet, der erhalten wird durch Umsetzung einer Nickel-(0)-Verbindung oder einer Verbindung, die in situ in eine Nickel-(0)-Verbindung übergeführt werden kann, mit Phosphorverbindungen der Formeln 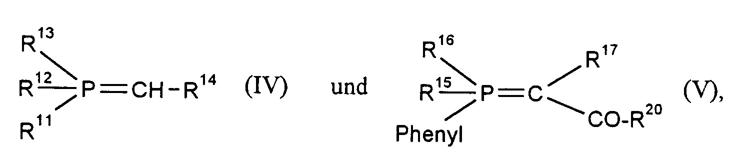 worin
R¹¹, R¹² und R¹³ unabhängig voneinander C₁-C₈-Alkyl, Phenyl oder Benzyl bedeuten,
R¹⁴ für Wasserstoff, C₁-C₈-Alkyl oder Phenyl steht,
R¹⁵, R¹⁶ und R¹⁷ unabhängig voneinander C₁-C₈-Alkyl oder Phenyl bedeuten, wobei R¹⁷ zusätzlich Wasserstoff oder Acyl bedeuten kann, und
R²⁰ Phenyl oder C₁-C₄-Alkyl bedeutet,
oder eines Nickel-Katalysators, herstellbar durch Umsetzung einer Nickel-(0)-Verbindung oder einer Verbindung, die in situ in eine Nickel-(0)-Verbindung übergeführt werden kann, mit einem Addukt aus Benzochinon oder Maleinsäureanhydrid und einem Phosphin der Formel 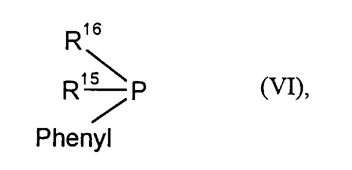 in der
R¹⁵ und R¹⁶ die genannte Bedeutung haben,
und einer Verbindung der Formel (IV).

5. Verfahren nach Anspruch 1, dadurch gekennzeichnet, daß 0,01 bis 100 mmol Nickel-Katalysator pro Mol Styrol-Derivat, bevorzugt 0,1 bis 10 mmol Nickel-Katalysator, besonders bevorzugt 0,2 bis 5 mmol Nickel-Katalysator eingesetzt werden.

6. Verfahren nach Anspruch 1, dadurch gekennzeichnet, daß ein Styrol-Derivat der Formel 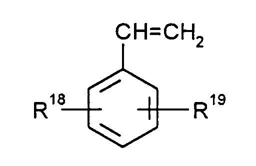 eingesetzt wird, in der
R¹⁸ Wasserstoff, C¹-C⁴-Alkyl, C₂-C₄-Alkenyl, C₂-C₇-Acyl, Fluor, Chlor oder Brom und
R¹⁹ Wasserstoff, C₁-C₄-Alkyl, Vinyl oder Chlor bedeuten.

7. Verfahren nach Anspruch 6, dadurch gekennzeichnet, daß ein Styrol-Derivat der Formel 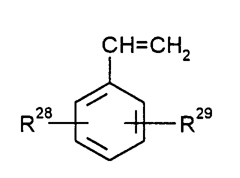 eingesetzt wird, in der
R²⁸ Wasserstoff, Methyl, Ethyl, i-Butyl, C₂-C₇-Acyl, Vinyl oder Chlor und
R²⁹ Wasserstoff, Vinyl, Methyl oder Chlor bedeuten.

8. Verfahren nach Anspruch 1, dadurch gekennzeichnet, daß bei einer Temperatur von 30 bis 160°C, bevorzugt 40 bis 120°C, besonders bevorzugt 50 bis 100°C, gearbeitet wird.

9. Mischungen von Verbindungen der Formel 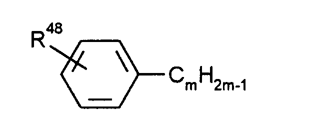 worin
R⁴⁸ Vinyl oder Benzoyl und m eine Zahl von 4 bis 204 bedeuten mit Ausnahme von Buten-(2)-yl-styrol, Buten-(3)-yl-styrol, Hexen-(2)-yl-styrol und Verbindungen, in denen R⁴⁸ Benzoyl bedeutet und m den Wert 4 annimmt.

10. Mischungen nach Anspruch 9, wobei m eine Zahl von 4 bis 64 bedeutet mit Ausnahme von Buten-(2)-yl-styrol, Buten-(3)-yl-styrol, Hexen-(2)-yl-styrol und Verbindungen, in denen R⁴⁸ Benzoyl bedeutet und m den Wert 4 annimmt.

## Claims

1. Process for the preparation of mixtures of compounds of the formula 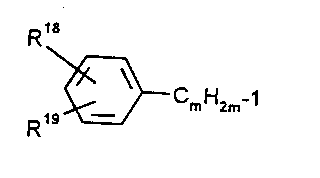 in which
R¹⁸ denotes hydrogen, C₁-C₄-alkyl, C₂-C₄-alkenyl, C₂-C₇-acyl, fluorine, chlorine or bromine,
R¹⁹ denotes hydrogen, C₁-C₄-alkyl, vinyl or chlorine and
m denotes 4 to 204,
by reaction of a compound of the formula 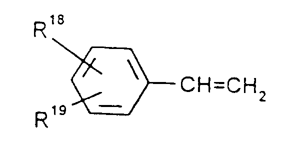 in which
R¹⁸ and R¹⁹ have the meaning defined above,
with ethylene in the presence of a nickel catalyst which carries a phosphorus-oxygen ligand and contains the structural unit, 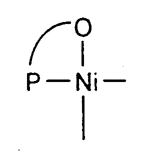 at a temperature of 20 to 160°C and an ethylene pressure of 1 to 200 bar.

2. Process according to Claim 1, characterised in that the reaction is carried out in the presence of a nickel catalyst which is obtained by reaction of a nickel(0) compound, or a compound which can be converted in situ to a nickel(0) compound, with a phosphorus compound of the formula 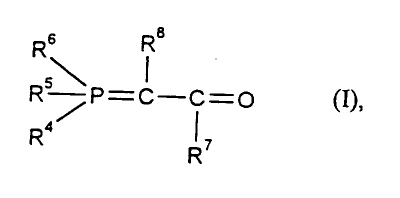 in which
R⁴, R⁵, R⁶, R⁷ and R⁸ independently of one another denote straight-chain or branched C₁-C₂₀-alkyl, C₂-C₂₀-alkenyl, C₁-C₂₀-alkoxy, C₃-C₈-cycloalkyl, C₆-C₁₂-aryl, C₆-C₁₂-aryloxy, C₇-C₁₅-aralkyl or C₇-C₁₅-aralkoxy, where
R⁷ can additionally denote hydrogen and
R⁸ can additionally denote hydrogen, acyl or sulphonate
or nickel catalysts which can be prepared by reaction of a nickel(0) compound, or a compound which can be converted in situ to a nickel(0) compound, with an adduct of a quinoid compound or maleic anhydride and a phosphine of the formula 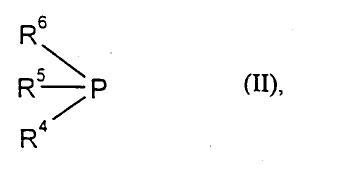 in which
R⁴, R⁵ and R⁶ have the meaning mentioned.

3. Process according to Claim 2, characterised in that, for the preparation of the nickel catalyst from a nickel(0) compound or a compound which can be converted in situ into a nickel(0) compound, and from a compound of the formula (I) or from an adduct of a quinoid compound or maleic anhydride and a compound of the formula (II), a compound of the formula 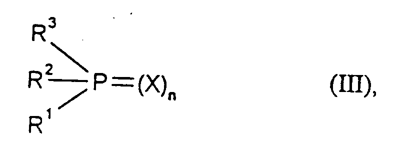 is additionally used as a starting material in which
R¹, R² and R³ independently of one another denote straight-chain or branched C₁-C₂₀-alkyl, C₁-C₂₀-alkoxy, C₃-C₈-cycloalkyl, C₂-C₂₀-alkenyl, di-(C₁-C₄-alkyl)-amino, C₆-C₁₂-aryl, C₆-C₁₂-aryloxy, C₇-C₁₅-aralkyl or C₇-C₁₅-aralkoxy,
X denotes doubly bonded oxygen, the doubly bonded group NR⁹ or the doubly bonded group 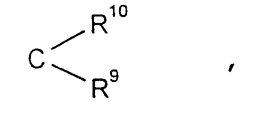 where
R⁹ and R¹⁰ independently of one another denote hydrogen, silyl, acyl, chlorophenyl, nitrophenyl, C₁-C₆-alkylphenyl, cyano, phenyl-C₂-C₆-alkenyl or R¹, and
n assumes the value zero or one.

4. Process according to Claim 3, characterised in that the reaction is carried out in the presence of a nickel catalyst which is obtained by reaction of a nickel(0) compound, or a compound which can be converted into a nickel(0) compound in situ, with phosphorus compounds of the formulae 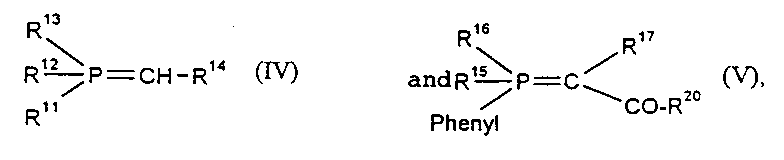 in which
R¹¹, R¹² and R¹³ independently of one another denote C₁-C₈-alkyl, phenyl or benzyl,
R¹⁴ represents hydrogen, C₁-C₈-alkyl or phenyl,
R¹⁵, R¹⁶ and R¹⁷ independently of one another denote C₁-C₈-alkyl or phenyl, where R¹⁷ can additionally denote hydrogen or acyl, and
R²⁰ denotes phenyl or C₁-C₄-alkyl,
or a nickel catalyst which can be prepared by reaction of a nickel(0) compound, or a compound which can be converted in situ into a nickel(0) compound, with an adduct of benzoquinone or maleic anhydride and a phosphine of the formula 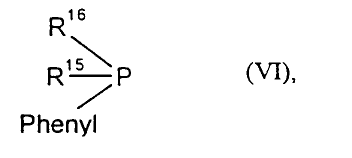 in which
R¹⁵ and R¹⁶ have the meaning mentioned,
and a compound of the formula (IV).

5. Process according to Claim 1, characterised in that 0.01 to 100 mmol of nickel catalyst are employed per mol of styrene derivative, preferably 0.1 to 10 mmol of nickel catalyst, particularly preferably 0.2 to 5 mmol of nickel catalyst.

6. Process according to Claim 1, characterised in that a styrene derivative of the formula 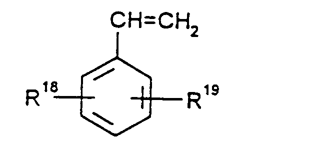 is employed in which
R¹⁸ is hydrogen, C₁-C₄-alkyl, C₂-C₄-alkenyl, C₂-C₇-acyl, fluorine, chlorine or bromine and
R¹⁹ is hydrogen, C₁-C₄-alkyl, vinyl or chlorine.

7. Process according to Claim 6, characterised in that a styrene derivative of the formula 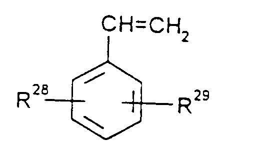 is employed in which
R²⁸ is hydrogen, methyl, ethyl, i-butyl, C₂-C₇-acyl, vinyl or chlorine and
R²⁹ is hydrogen, vinyl, methyl or chlorine.

8. Process according to Claim 1, characterised in that it is carried out at a temperature of 30 to 160°C, preferably 40 to 120°C, particularly preferably 50 to 100°C.

9. Mixtures of compounds of the formula 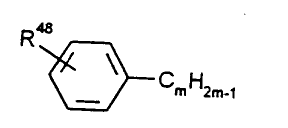 in which
R⁴⁸ denotes vinyl or benzoyl and m denotes a number from 4 to 204, with the exception of buten-(2)-yl-styrene, buten-(3)-yl-styrene, hexen-(2)-yl-styrene and compounds in which R⁴⁸ denotes benzoyl and m assumes the value 4.

10. Mixtures according to Claim 9, m denoting a number from 4 to 64, with the exception of buten-(2)-yl-styrene, buten-(3)-yl-styrene, hexen-(2)-yl-styrene and compounds in which R⁴⁸ denotes benzoyl and m assumes the value 4.

## Revendications

1. Procédé de préparation de mélanges de composés de formule 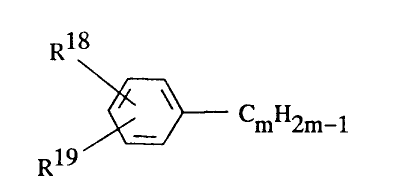 dans laquelle
R¹⁸ représente l'hydrogène, un groupe alkyle en C₁-C₄, alcényle en C₂-C₄, acyle en C₂-C₇, le fluor, le chlore ou le brome,
R¹⁹ représente l'hydrogène, un groupe alkyle en C₁-C₄, vinyle ou le chlore et
m est un nombre allant de 4 à 204,
par réaction d'un composé de formule 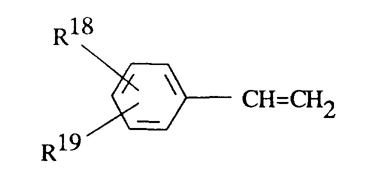 dans laquelle
R¹⁸ et R¹⁹ ont les significations indiquées ci-dessus,
avec l'éthylène en présence d'un catalyseur au nickel portant un ligand phosphoré et oxygéné et contenant le motif de structure 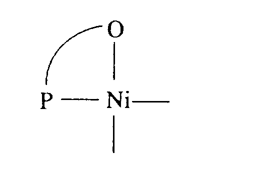 à une température de 20 à 160°C et une pression d'éthylène de 1 à 200 bar.

2. Procédé selon la revendication 1, caractérisé en ce que l'on opère en présence d'un catalyseur au nickel obtenu par réaction d'un dérivé de nickel-(0) ou d'un composé pouvant être converti in situ en un dérivé du nickel-(0), avec un dérivé du phosphore répondant à la formule 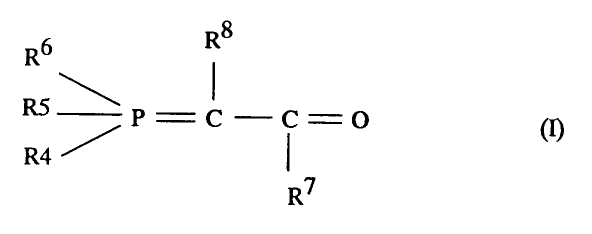 dans laquelle
R⁴, R⁵, R⁶, R⁷ et R⁸ représentent chacun, indépendamment les uns des autres, un groupe alkyle à chaîne droite ou ramifiée en C₁-C₂₀, alcényle en C₂-C₂₀, alcoxy C₁-C₂₀, cycloalkyle en C₃-C₈, aryle en C₆-C₁₂, aralkyle en C₇-C₁₅ ou aralcoxy en C₇-C₁₅,
R⁷ pouvant en outre représenter l'hydrogène,
R⁸ pouvant en outre représenter l'hydrogène, un radical acyle ou un groupe sulfonate,
ou de catalyseurs au nickel préparés par réaction d'un dérivé du nickel-(0) ou d'un composé pouvant être converti in situ en un dérivé du nickel-(0), avec un adduct d'un dérivé quinoïde ou de l'anhydride maléique et d'une phosphine de formule 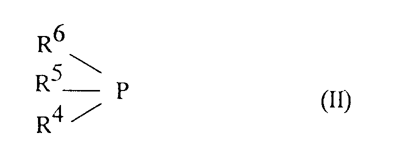 dans laquelle
R⁴, R⁵ et R⁶ ont les significations indiquées ci-dessus.

3. Procédé selon la revendication 2, caractérisé en ce que, pour la préparation du catalyseur au nickel, on part d'un dérivé du nickel-(0) ou d'un composé pouvant être converti in situ en un dérivé du nickel-(0), et d'un composé de formule (I) ou d'un adduct d'un dérivé quinoïde ou de l'anhydride maléique et d'un composé de formule (II), et en outre d'un composé de formule 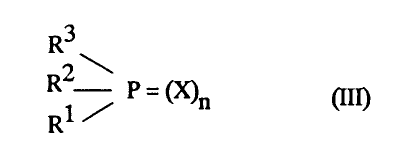 dans laquelle
R¹, R² et R³ représentent chacun, indépendamment les uns des autres, un groupe alkyle à chaîne droite ou ramifiée en C₁-C₂₀, alcoxy C₁-C₂₀, cycloalkyle en C₃-C₈, alcényle en C₂-C₂₀, di(alkyle en C₁-C₄)amino, aryle en C₆-C₁₂, aryloxy en C₆-C₁₂, aralkyle en C₇-C₁₅ ou aralcoxy en C₇-C₁₅,
X représente l'oxygène relié par une double liaison, le groupe NR⁹ relié par une double liaison ou le groupe 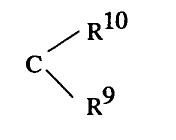 relié par une double liaison,
R⁹ et R¹⁰ représentant chacun, indépendamment l'un de l'autre, l'hydrogène, un groupe silyle, acyle, chlorophényle, nitrophényle, (alkyle en C₁-C₆)phényle, cyano, phényl-alcényle en C₂-C₆ ou R¹ et
n est égal à 0 ou 1.

4. Procédé selon la revendication 3 caractérisé en ce que l'on opère en présence d'un catalyseur au nickel obtenu par réaction d'un dérivé du nickel-(0) ou d'un composé qui peut être converti in situ en un dérivé du nickel-(0), avec des dérivés du phosphore de formules 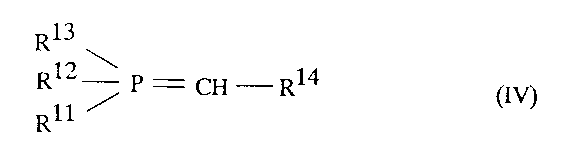 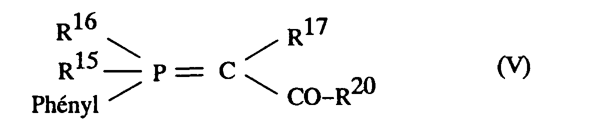 dans lesquelles
R¹¹, R¹² et R¹³ représentent chacun, indépendamment les uns des autres, un groupe alkyle en C₁-C₈, phényle ou benzyle,
R¹⁴ représente l'hydrogène, un groupe alkyle en C₁-C₈ ou phényle,
R¹⁵, R¹⁶ et R¹⁷ représentent chacun, indépendamment les uns des autres, un groupe alkyle en C₁-C₈ ou phényle, R¹⁷ pouvant en outre représenter l'hydrogène ou un radical acyle et
R²⁰ représente un groupe phényle ou alkyle en C₁-C₄,
ou d'un catalyseur au nickel préparé par réaction d'un dérivé du nickel-(0) ou d'un composé qui peut être converti in situ en un dérivé du nickel-(0) avec un adduct de la benzoquinone ou de l'anhydride maléique et d'une phosphine de formule 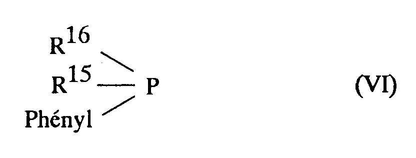 dans laquelle
R¹⁵ et R¹⁶ ont les significations indiquées ci-dessus,
et d'un composé de formule (IV).

5. Procédé selon la revendication 1, caractérisé en ce que l'on utilise de 0,01 à 100 mmol du catalyseur au nickel par mole du dérivé du styrène, de préférence 0,1 à 10 mmol du catalyseur au nickel et plus spécialement de 0,2 à 5 mmol du catalyseur au nickel.

6. Procédé selon la revendication 1, caractérisé en ce que l'on met en oeuvre un dérivé du styrène répondant à la formule 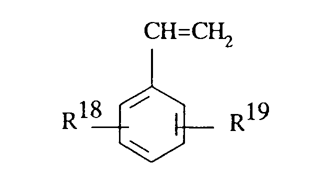 dans laquelle
R¹⁸ représente l'hydrogène, un groupe alkyle en C₁-C₄, alcényle en C₂-C₄, acyle en C₂-C₇, le fluor, le chlore ou le brome et
R¹⁹ représente l'hydrogène, un groupe alkyle en C₁-C₄, vinyle ou le chlore.

7. Procédé selon la revendication 6, caractérisé en ce que l'on met en oeuvre un dérivé du styrène de formule 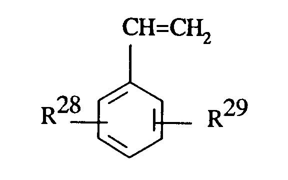 dans laquelle
R²⁸ représente l'hydrogène, un groupe méthyle, éthyle, isobutyle, acyle en C₂-C₇, vinyle ou le chlore et
R²⁹ représente l'hydrogène, un groupe vinyle, méthyle ou le chlore.

8. Procédé selon la revendication 1 caractérisé en ce que l'on opère à des températures de 30 à 160°C, de préférence de 40 à 120°C et plus spécialement de 50 à 100°C.

9. Mélanges de composés de formule 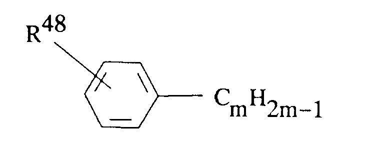 dans laquelle
R⁴⁸ représente un groupe vinyle ou benzoyle et
m est un nombre allant de 4 à 204, à l'exception du butène-(2)-yl-styrène, du butène-(3)-yl-styrène, de l'hexène-(2)-yl-styrène et des composés pour lesquels
R⁴⁸ représente un groupe benzoyle et m est égal à 4.

10. Mélanges selon la revendication 9, pour lesquels m est un nombre allant de 4 à 64, à l'exception du butène-(2)-yl-styrène, du butène-(3)-yl-styrène, de l'hexène-(2)-yl-styrène et des composés pour lesquels R⁴⁸ représente un groupe benzoyle et m est égal à 4.
